# EUROPEAN PATENT APPLICATION

(11) **EP 3 254 655 A1**
(43) Date of publication of application: **13.12.2017**
(21) Application number: 16201727.1
(22) Date of filing: 01.12.2016
(51) Int. Cl.: A61F 13/511, A61F 13/512, A61F 13/513, D04H 1/425, D04H 1/4266, D04H 3/007, D04H 3/011, D04H 5/03, D04H 5/06

(54) **TOP SHEET FOR PERSONAL CARE PRODUCT**

(30) Priority: 10.06.2016 IN 201621019950
(71) Applicant: Welspun India Limited, Mumbai 400013 (IN)
(72) Inventor: Goenka, Dipali, 400013 Mumbai (IN); Sahu, Pranay, 400013 Mumbai (IN)
(74) Representative: Avidity IP

(57) **Abstract**

A top sheet for an absorbent personal care product is a water permeable composite web produced by hydro-entangling a web of chemical free natural and / or cellulosic staple fibers and / or blend of natural and cellulosic staple fibers and a web of man-made continuous filament yarns such that a first side of the composite web exposes the chemical free natural and / or cellusoic staple fibers and / or blend of natural and cellulosic staple fibers and an opposite second side of the composite web has the man-made continuous filament yarns. The web of chemical free natural and / or cellulosic staple fibers and / or blend of natural and cellulosic staple fibers is hydrophilic and the web of man-made continuous filament synthetic yarns is hydrophobic. The composite web weighs between about 20 gsm and about 40 gsm.

## Description

### FIELD OF THE INVENTION

The present invention relates generally to nonwoven fabrics and, more particularly, to composite non-woven fabrics.

### BACKGROUND

An absorbent personal care product, such as a diaper or sanitary feminine napkin, typically includes, without limitation, an outer cover, or backsheet, a liquid permeable bodyside liner, or topsheet, positioned in facing relation with the backsheet, and an absorbent body, or liquid retention structure, located between the backsheet and the topsheet. Typically, a topsheet is sufficiently porous to be liquid-permeable, permitting liquid to readily penetrate through its thickness to reach an absorbent body. The topsheet layer is typically employed to help isolate the wearer's skin from liquids held in the absorbent body.

Conventionally, top sheets are formed from spunbond nonwoven material, thermo-bond nonwoven material, or perforated films, such as polyethylene. During use of an absorbent personal care product, the top sheet is directly next to the wearer's skin and, unfortunately, may cause skin allergies and other irritations as a result of the chemical composition of the top sheet. Efforts have been made to produce top sheets from chemical-free materials, such as spunlace nonwoven material made from 100% bleached cotton fibers. Unfortunately, chemical-free spunlace materials can be expensive to manufacture. In addition, spunlace materials may have lower strength and/or stability as compared with conventional top sheet materials.

### SUMMARY

It should be appreciated that this Summary is provided to introduce a selection of concepts in a simplified form, the concepts being further described below in the Detailed Description. This Summary is not intended to identify key features or essential features of this disclosure, nor is it intended to limit the scope of the invention.

According to some embodiments of the present invention, a nonwoven fabric includes a first side of chemical free natural and / or cellulosic staple fibers and / or blend of natural and cellulosic staple fibers and an opposite second side of man-made continuous filament yarns, and weighs between about 20 gsm and about 40 gsm. The nonwoven fabric is a composite web produced by hydro-entangling a web of the chemical free natural and / or cellulosic staple fibers and / or blend of natural and cellulosic staple fibers and a web of the man-made continuous filament yarns. In some embodiments, the web of chemical free natural and / or cellulosic staple fibers and / or blend of natural and cellulosic staple fibers weighs at least about 10 gsm and is hydrophilic and the web of man-made continuous filament synthetic yarns weighs at least about 10 gsm and is hydrophobic. In some embodiments, the composite web is water-permeable.

The chemical free natural and / or cellulosic staple fibers and / or blend of natural and cellulosic staple fibers are chemical-free (i.e., untreated) and may include, but are not limited to, cotton, grey cotton, bamboo, viscose, modal, tencel, silk, and wool. In some embodiments, the web of chemical free natural and / or cellulosic staple fibers and / or blend of natural and cellulosic staple fibers is a blend of two or more fibers, such as cotton, grey cotton, bamboo, viscose, modal, tencel, silk, and wool. In some embodiments, the man-made continuous filament yarns are chemical-free (i.e., untreated) polyester or polypropylene yarns. In some embodiments, the man-made continuous filament yarns comprise bi-component fibers.

According to some embodiments of the present invention, a top sheet for an absorbent personal care product, such as a diaper, sanitary napkin, etc., is provided. The top sheet is a liquid-permeable composite web produced by hydro-entangling a web of chemical free natural and / or cellulosic staple fibers and / or blend of natural and cellulosic staple fibers and a web of man-made continuous filament yarns such that a first side of the composite web exposes the chemical free natural and / or cellulosic staple fibers and / or blend of natural and cellulosic staple fibers and an opposite second side of the composite web exposes the man-made continuous filament yarns. The web of chemical free natural and / or cellulosic staple fibers and / or blend of natural and cellulosic staple fibers is hydrophilic and the web of man-made continuous filament synthetic yarns is hydrophobic. In some embodiments, the composite web weighs between about 20 gsm and about 40 gsm. In some embodiments, the web of chemical free natural and / or cellulosic staple fibers and / or blend of natural and cellulosic staple fibers weighs at least about 10 gsm, and the web of man-made continuous filament synthetic yarns weighs at least about 10 gsm.

The chemical free natural and / or cellulosic staple fibers and / or blend of natural and cellulosic staple fibers for the top sheet may include, but are not limited to, cotton, grey cotton, bamboo, viscose, modal, tencel, silk, and wool. In some embodiments, the web of chemical free natural and / or cellulosic staple fibers and / or blend of natural and cellulosic staple fibers is a blend of two or more of these fibers. In some embodiments, the man-made continuous filament yarns for the top sheet are polyester or polypropylene yarns. In some embodiments, the man-made continuous filament yarns for the top sheet are bi-component fibers.

According to other embodiments of the present invention, a method of producing a top sheet for an absorbent personal care product, such as a diaper, sanitary napkin, etc., includes hydro-entangling a web of chemical free natural and / or cellulosic staple fibers and / or blend of natural and cellulosic staple fibers and a web of man-made continuous filament yarns to produce a composite web such that a first side of the composite web exposes the chemical free natural and / or cellulosic staple fibers and / or blend of natural and cellulosic staple fibers and an opposite second side of the composite web exposes the man-made continuous filament yarns. In some embodiments, the web of chemical free natural and / or cellulosic staple fibers and / or blend of natural and cellulosic staple fibers weighs at least about 10 gsm, the web of man-made continuous filament synthetic yarns weighs at least about 10 gsm, and the composite web weighs between about 20 gsm and about 40 gsm.

In some embodiments, the method also includes forming apertures in the composite web and/or embossing the composite web to form patterns and/or characters thereon.

It is noted that aspects of the invention described with respect to one embodiment may be incorporated in a different embodiment although not specifically described relative thereto. That is, all embodiments and/or features of any embodiment can be combined in any way and/or combination. Applicant reserves the right to change any originally filed claim or file any new claim accordingly, including the right to be able to amend any originally filed claim to depend from and/or incorporate any feature of any other claim although not originally claimed in that manner. These and other objects and/or aspects of the present invention are explained in detail below.

### BRIEF DESCRIPTION OF THE DRAWINGS

The accompanying drawings, which form a part of the specification, illustrate some exemplary embodiments. The drawings and description together serve to fully explain the exemplary embodiments.
Fig. 1 is a perspective view of a nonwoven, composite web produced by hydro-entangling a web of chemical free natural and / or cellulosic staple fibers and / or blend of natural and cellulosic staple fibers and a web of man-made continuous filament yarns, according to some embodiments of the present invention.
Fig. 2 is a side view of the composite web of Fig. 1.
Fig. 3 is an enlarged side view of the composite web of Fig. 1 illustrating the hydro-entanglement of the two webs.
Fig. 4 illustrates a method of producing the composite web of Figs. 1-3, according to some embodiments of the present invention.
Fig. 5 is a flow chart of operations for producing the composite web of Figs. 1-3, according to some embodiments of the present invention.
Fig. 6 is an enlarged, partial cross-sectional view of an absorbent personal care product utilizing the composite web of Fig. 1 as a topsheet, according to some embodiments of the present invention.

### DETAILED DESCRIPTION

The present invention will now be described more fully hereinafter with reference to the accompanying figures, in which embodiments of the invention are shown. This invention may, however, be embodied in many different forms and should not be construed as limited to the embodiments set forth herein. Like numbers refer to like elements throughout. In the figures, certain components or features may be exaggerated for clarity, and broken lines illustrate optional features or elements unless specified otherwise. In addition, the sequence of operations (or steps) is not limited to the order presented in the figures and/or claims unless specifically indicated otherwise. Features described with respect to one figure or embodiment can be associated with another embodiment or figure although not specifically described or shown as such.

It will be understood that when a feature or element is referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments.

The terminology used herein is for the purpose of describing particular embodiments only and is not intended to be limiting of the invention. As used herein, the singular forms "a", "an" and "the" are intended to include the plural forms as well, unless the context clearly indicates otherwise.

As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of an article/device in use or operation in addition to the orientation depicted in the figures. For example, if an article/device in the figures is inverted, elements described as "under" or "beneath" other elements or features would then be oriented "over" the other elements or features. Thus, the exemplary term "under" can encompass both an orientation of over and under. The article/device may be otherwise oriented

(rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

It will be understood that although the terms first and second are used herein to describe various features or elements, these features or elements should not be limited by these terms. These terms are only used to distinguish one feature or element from another feature or element. Thus, a first feature or element discussed below could be termed a second feature or element, and similarly, a second feature or element discussed below could be termed a first feature or element without departing from the teachings of the present invention. Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

As used herein, the terms "comprise", "comprising", "comprises", "include", "including", "includes", "have", "has", "having", or variants thereof, are open-ended, and include one or more stated features, elements, steps, components or functions but does not preclude the presence or addition of one or more other features, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

The term "about", as used herein with respect to a value or number, means that the value or number can vary more or less, for example by +/- 20%, +/- 10%, +/- 5%, +/- 1%, +/- 0.5%, +/-0.1 %, etc.

As used herein, the terms "nonwoven fabric, "nonwoven web" and "nonwoven material" are interchangeable and refer to a web having a structure of individual fibers, filaments or threads which are interlaid, but not in a regular or identifiable manner such as those in a knitted fabric or films that have been fibrillated. Nonwoven webs or materials may be formed from many processes such as, for example, meltblowing processes, spunbonding processes, and bonded carded web processes. The basis weight of nonwoven webs or materials is expressed in ounces of material per square yard (osy) or grams per square meter (gsm), and the fiber diameters are expressed in microns.

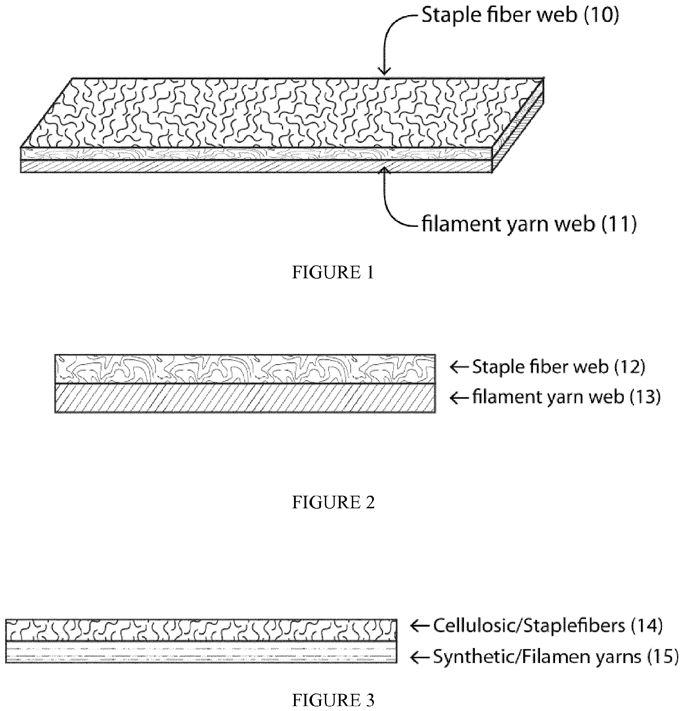

Referring to Figs. 1-3, a nonwoven fabric 10, according to some embodiments of the present invention and that may be utilized as a top sheet for an absorbent personal care product, such as a diaper, sanitary napkin, etc., is illustrated. The fabric has a first side 10a of exposed chemical free natural and / or cellulosic staple fibers and / or blend of natural and cellulosic staple fibers 12 and an opposite second side 10b of man-made continuous filament yarns 14.

The nonwoven fabric 10 is a composite web produced by hydro-entangling a web 16 of the chemical free natural and / or cellulosic staple fibers and / or blend of natural and cellulosic staple fibers 12 and a web 18 of the man-made continuous filament yarns 14. Fig. 3 illustrates the mechanical entanglement of the fibers of the two webs 16, 18 at an interface 20 thereof. The web 16 of staple fibers 12 is configured to provide comfort to a person and is hydrophilic. The web 18 of continuous filament synthetic yarns 14 provides strength to the composite web and is hydrophobic. The combination of the two webs 16, 18 provides a cost effective, strong structure as compared with conventional top sheet products.

In some embodiments, the web 16 of chemical free natural and / or cellulosic staple fibers and / or blend of natural and cellulosic staple fibers 12 weighs at least about 10 gsm and the web 18 of the man-made continuous filament synthetic yarns 14 weighs at least about 10 gsm. In some embodiments, the composite web 10 weighs between about 20 gsm and about 40 gsm. In some embodiments, the composite web 10 is water-permeable. For example, when utilized as a top sheet of an absorbent personal care product, liquid/moisture can pass through the top sheet to an adjacent absorbent element. In some embodiments, the staple fibers 12 are between about 0.7 denier and about 4 denier. In some embodiments, the staple fibers 12 have a length between about 12mm and about 48mm.

The chemical free natural and / or cellulosic staple fibers and / or blend of natural and cellulosic staple fibers 12 may include, but are not limited to, cotton, grey cotton, bamboo, viscose, modal, tencel, silk, and wool. In some embodiments, the web 16 of chemical free natural and / or cellulosic staple fibers and / or blend of natural and cellulosic staple fibers 12 is a blend of two or more of these fibers. In some embodiments, the man-made continuous filament yarns 14 are polyester or polypropylene yarns, for example, 100% polyester or 100% polypropylene. In some embodiments, the man-made continuous filament yarns 14 are bi-component synthetic fibers.

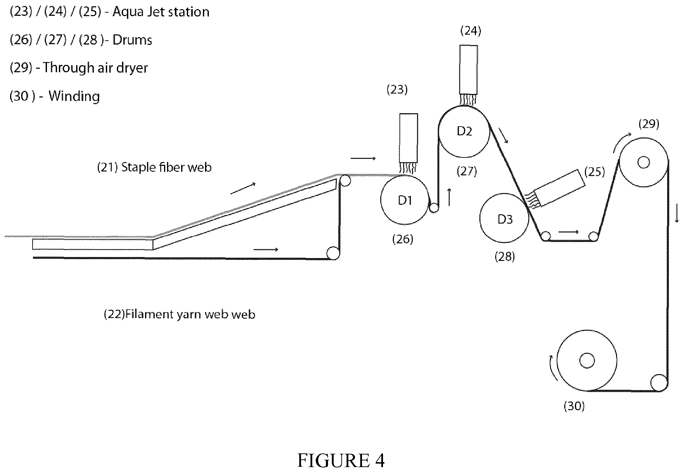

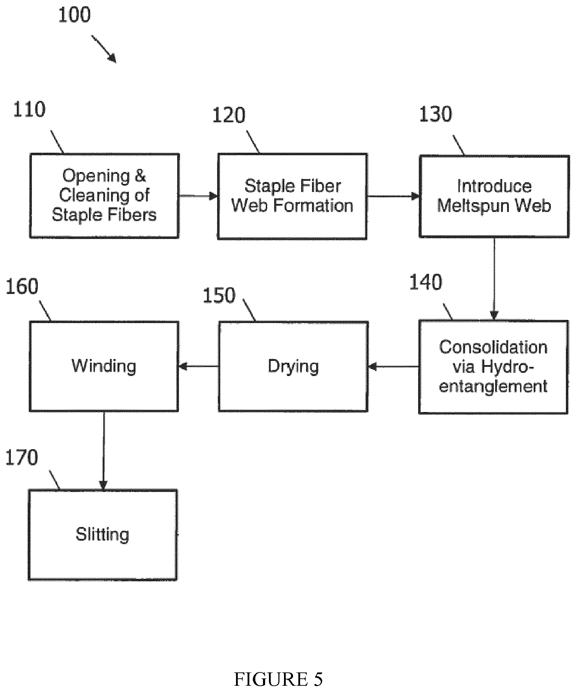

Referring to Figs. 4 and 5, methods of making a top sheet for an absorbent personal care product, such as a diaper, sanitary napkin, etc., are illustrated. Referring initially to Fig. 4, a web 16 of chemical free natural and / or cellulosic staple fibers and / or blend of natural and cellulosic staple fibers 12 and a web 18 of the continuous filament synthetic yarns 14 are hydroentangled at three aqua jet stations, 23, 24, 25 located at respective drums 26, 27, 28. These aqua jet stations 23, 24, 25 utilize jets of water under pressure to entangle the fibers of the two webs 16, 18 and mechanically bond the two webs 16, 18 together. Downstream from the third aqua jet station 25, the hydro-entangled composite web 10 is fed through one or more air dryers 29 and then wound into a roll 30.

Fig. 5 is a flow chart illustrating operations for a method 100 of making a top sheet for an absorbent personal care product, according to some embodiments of the present invention. Initially, the chemical free natural and / or cellulosic staple fibers and / or blend of natural and cellulosic staple fibers 12 are opened and cleaned (Block 110) and formed into a web 16 (Block 120), as would be understood by one skilled in the art. A web 18 of continuous synthetic fibers 14 is then positioned adjacent the web 16 of staple fibers (Block 130) and the two webs 16, 18 are consolidated into a composite web 10 via hydro-entanglement (Block 140). The hydro-entangled composite web 10 is subject to drying operations (Block 150) and wound into rolls (Block160). Various additional operations may be performed on the composite web 10, such as slitting operations (Block 170), for example to produce apertures in the composite web 10. In addition, the composite web 10 can be embossed to form patterns and/or characters thereon. Moreover, the composite web 10 can be produced so as to have a plain appearance or can be produced to have various colors, also.

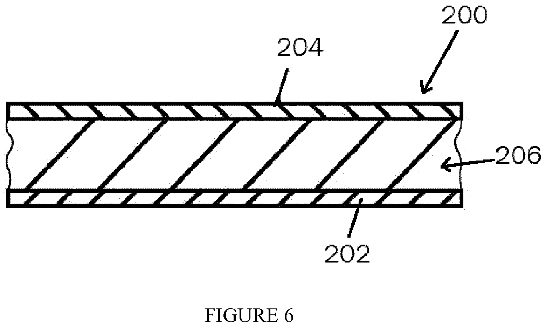

Referring to Fig. 6, an absorbent personal care product 200 utilizing the composite web 10 of Figs. 1-4 as a topsheet, is illustrated. The illustrated absorbent personal care product 200 includes a backsheet 202 and a liquid-permeable topsheet 204 (i.e., the composite web 10 of Figs. 1-4) positioned in facing relation with the backsheet 202. An absorbent body 206 is sandwiched between the backsheet 202 and the topsheet 204.

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the teachings and advantages of this invention. Accordingly, all such modifications are intended to be included within the scope of this invention as defined in the claims.

## Claims

1. A nonwoven fabric, comprising a first side of exposed chemical free natural and / or cellulosic staple fibers and / or blend of natural and cellulosic staple fibers and an opposite second side of man-made continuous filament yarns, wherein the nonwoven fabric weighs between about 20 gsm and about 40 gsm.

2. The nonwoven fabric of Claim 1, wherein the nonwoven fabric is a composite web produced by hydro-entangling a web of the chemical free natural and / or cellulosic staple fibers and / or blend of natural and cellulosic staple fibers and a web of the man-made continuous filament yarns.

3. The nonwoven fabric of Claim 1, wherein the chemical free natural and / or cellulosic staple fibers and / or blend of natural and cellulosic staple fibers comprise chemical-free fibers selected from the group consisting of cotton, grey cotton, bamboo, viscose, modal, tencel, silk, and wool.

4. The nonwoven fabric of Claim 1, wherein the man-made continuous filament yarns comprise polyester or polypropylene, and optionally comprise bi-component fibers.

5. The nonwoven fabric of Claim 2, wherein the web of chemical free natural and / or cellulosic staple fibers and / or blend of natural and cellulosic staple fibers is hydrophilic, and wherein the web of man-made continuous filament synthetic yarns is hydrophobic.

6. The nonwoven fabric of Claim 2, wherein composite web is water-permeable.

7. The nonwoven fabric of Claim 2, wherein the web of chemical free natural and / or cellulosic staple fibers and / or blend of natural and cellulosic staple fibers weighs at least about 10 gsm, and wherein the web of man-made continuous filament synthetic yarns weighs at least about 10 gsm.

8. A top sheet for an absorbent personal care product, the top sheet comprising:
a liquid-permeable composite web produced by hydro-entangling a web of chemical free natural and / or cellulosic staple fibers and / or blend of natural and cellulosic staple fibers and a web of man-made continuous filament yarns, wherein the web of chemical free natural and / or cellulosic staple fibers and / or blend of natural and cellulosic staple fibers is hydrophilic, wherein the web of man-made continuous filament synthetic yarns is hydrophobic, wherein the composite web weighs between about 20 gsm and about 40 gsm, wherein a first side of the composite web exposes the chemical free natural and / or cellulosic staple fibers and / or blend of natural and cellulosic staple fibers and wherein an opposite second side of the composite web comprises the man-made continuous filament yarns.

9. The top sheet of Claim 8, wherein the chemical free natural and / or cellulosic staple fibers and / or blend of natural and cellulosic staple fibers comprise chemical-free fibers selected from the group consisting of cotton, grey cotton, bamboo, viscose, modal, tencel, silk, and wool.

10. The top sheet of Claim 8, wherein the man-made continuous filament yarns comprise polyester or polypropylene, and optionally comprise bi-component fibers.

11. The top sheet of Claim 8, wherein the web of chemical free natural and / or cellulosic staple fibers and / or blend of natural and cellulosic staple fibers weighs at least about 10 gsm, and wherein the web of man-made continuous filament synthetic yarns weighs at least about 10 gsm.

12. A method of producing a top sheet for an absorbent personal care product, the method comprising hydro-entangling a web of chemical free natural and / or cellulosic staple fibers and / or blend of natural and cellulosic staple fibers and a web of man-made continuous filament yarns to produce a composite web such that a first side of the composite web exposes the chemical free natural and / or cellulosic staple fibers and / or blend of natural and cellulosic staple fibers and an opposite second side of the composite web comprises the man-made continuous filament yarns.

13. The method of Claim 12, wherein the web of chemical free natural and / or cellulosic staple fibers and / or blend of natural and cellulosic staple fibers weighs at least about 10 gsm, and wherein the web of man-made continuous filament synthetic yarns weighs at least about 10 gsm.

14. The method of Claim 12, wherein the composite web weighs between about 20 gsm and about 40 gsm.

15. The method of Claim 12, further comprising forming apertures in the composite web.

16. The method of Claim 12, further comprising embossing the composite web to form patterns and/or characters thereon.
